(19) Europäisches Patentamt
European Patent Office
Office européen des brevets

(11) Publication number: **0 042 570**
**B1**

# (12) EUROPEAN PATENT SPECIFICATION

(45) Date of publication of patent specification: **19.10.83**

(51) Int. Cl.³: **C 07 H 17/08,**
A 61 K 31/70

(21) Application number: **81104611.9**

(22) Date of filing: **15.06.81**

## (54) 20-Deoxyrosaramicin derivatives, process for their preparation and pharmaceutical compositions containing them.

(30) Priority: **23.06.80 US 161947**

(43) Date of publication of application:
**30.12.81 Bulletin 81/52**

(45) Publication of the grant of the patent:
**19.10.83 Bulletin 83/42**

(84) Designated Contracting States:
**BE CH DE FR GB IT LI LU NL SE**

(56) References cited:
FR - A - 2 302 102
US - A - 4 056 616
US - A - 4 161 523

(73) Proprietor: **SCHERING CORPORATION**
**2000 Galloping Hill Road**
**Kenilworth, New Jersey 07033 (US)**

(72) Inventor: **Ganguly, Ashit kumar**
**96 Cooper Avenue**
**Upper Montclair New Jersey 07043 (US)**
Inventor: **Liu, Yi-Tsung**
**19 Schuyler Street**
**Parsippany New Jersey 07054 (US)**

(74) Representative: **Antony, Fritz, Dr. et al,**
**P.O. Box 601 Winkelriedstrasse 35**
**CH-6002 Lucerne (CH)**

**EP 0 042 570 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 20-deoxyrosaramicin derivatives, process for their preparation and pharmaceutical compositions containing them

This invention relates to novel 20-deoxy-20-imino-rosaramicin derivatives, a process for their preparation and pharmaceutical compositions containing them. These new compounds possess potent and broad spectrum antibacterial activity.

Rosaramicin (previously named rosamicin) is known from US patent 4161523 and 12,13-desepoxy-12,13-dehydro rosaramicin from US patent 4056616 and FR-A-2-2302102. A new group of compounds has been developed by replacing the group of position 20 of the known compounds by the group CH=N—Z (defined further below).

The new compounds according to the present invention are compounds of the general formula

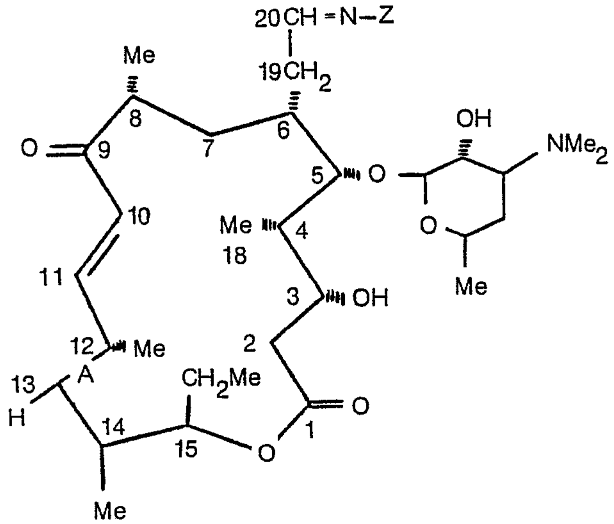

I

wherein Me is methyl;
A represents a double bond between the carbon atoms of positions 12 and 13 or, together with the carbon atoms of positions 12 and 13, an oxirane ring;
Z is a group of formula IIa or IIb,

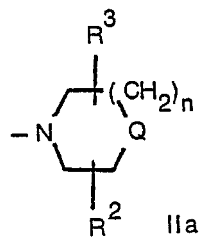

IIa

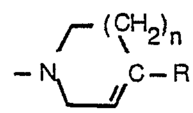

II b

wherein Q is CRR[1], NR, O, S, $SO_2$,

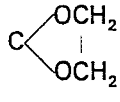

CROR[1], CROC(O)R[1], CHCOOR, CHCONRR[1] or CRCN; wherein R and R[1] independent of each other represent hydrogen, alkyl containing 1 to 8 carbon atoms, aralkyl containing 7 to 13 carbon atoms or aryl containing 6 to 10 carbon atoms, the aryl groups being unsubstituted or being substituted by halogen, trifluoromethyl, alkoxy containing 1 to 5 carbon atoms or alkylcarbonyl containing 1 to 5 carbon atoms or alkyl containing 1 to 5 carbon atoms; and when one of R and R[1] is hydrogen the other one can also be hydroxyalkyl containing 1 to 8 carbon atoms;
$R^2$ and $R^3$ independent of each other are hydrogen, alkyl containing 1 to 8 carbon atoms, oxo or thioxo;
n is zero, 1 or 2;
and the pharmaceutically acceptable acid addition salts thereof.

Certain groups of compounds are of particular interest:

—) Compounds of formula 1, wherein Z is a group of formula (IIa), wherein $R^2$ and $R^3$ are hydrogen, especially compounds wherein Q is CRR[1], NR, O, S, $SO_2$,

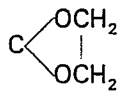 ,

CROR$^1$, CROC(O)R$^1$, CHCOOR, CHCONRR$^1$ or CRCN; wherein R and R$^1$ independent of each other represent hydrogen, alkyl containing 1 to 5 carbon atoms, aralkyl containing 7 to 13 carbon atoms or aryl containing 6 carbon atoms, the aryl groups being unsubstituted or being substituted by fluoro, chloro, trifluoromethyl, methoxy, acetyl or methyl, and when one of R and R$^1$ is hydrogen the other one can also be hydroxyalkyl containing 1 or 2 carbon atoms. Of particular interest are compounds of formula I, wherein n is zero or one, especially compounds of formula I wherein Me and A are defined as above, Z is a group of formula (IIa), wherein R$^2$ and R$^3$ are hydrogen, Q is $NCH_3$, S, $SO_2$,

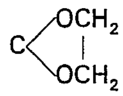

or CHOH and n is one.

—) Compounds of formula I, wherein Z is a group of formula (IIa), Q is $CH_2$, R$^2$ and R$^3$ independent of each other are alkyl containing 1 to 8 carbon atoms, oxo or thioxo, n is zero or one and Me and A are defined as above.

—) Compounds of formula I, wherein Z is a group of formula (IIb), n is one and Me, A and R are as defined above.

The compounds of this invention are broad spectrum antibacterial agents exhibiting significant activity against numerous strains of *Staphylococcus, Streptococcus, Enterobacter, Eschericia Coli, Bacillus, Pseudomonas, Clostridium* and *Corynebacterium.* Included among these bacteria are *Strep. pyogenes C, Staph 209P, Staph. Wood, Strept Karipedes, Enterobacter 1022, E. Coli 10536,* and *Klebsiella Rahal 3.* Some of the foregoing bacteria are clinical isolates obtained from patients having active infections.

The compounds of this invention may be used in the treatment of mammals having a bacterial infection. The compounds may also be used for in vitro purposes as disinfectants for laboratory glassware, dental and medical equipment.

In order to elicit an antibacterial effect, the compounds may be administered orally, topically, intramuscularly or intravenously. Administration may be effected by the use of the usual pharmaceutical formulations such as for example tablets, capsules, elixirs and injectable suspensions and solutions, creams and ointments. Preferably the active compounds are admixed with non-toxic pharmaceutically acceptable carriers and excipients generally used in the art. The compounds of this invention may advantageously be administered at from about 5 mg to about 50 mg per kg per day in divided doses.

The compounds of this invention can be prepared by reacting rosaramicin or 12,13-desepoxy-12,13-dehydrorosaramicin, respectively, with a compound of the general formula

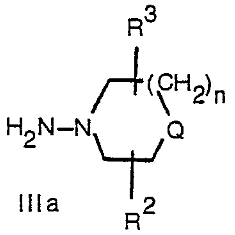

IIIa

or

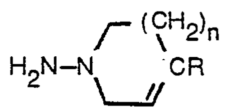

IIIb

wherein n, Q, R, R$^2$ and R$^3$ are defined as above for formula I. Preferably the reactants are dissolved in a non-aqueous solvent, preferably ethanol (especially absolute ethanol) and reacted at room temperature. The so obtained compounds of formula I are isolated in the free form or in the form of their pharmaceutically acceptable acid addition salts.

Pharmaceutically acceptable acid addition salts of this invention are derived from acids generally employed in the pharmaceutical art, including inorganic acids, such as for example sulfuric, phosphoric and hydrohalic (e.g. hydrochloric), and boric acid and carboxylic acids having 2 to 18 carbon atoms, such as for example acetic, propionic, valeric, stearic, tartaric, maleic, oxalic, malic, malonic, citric, oleic, palmitic, lauric, lactic, fumaric, succinic, cyclopropylcarboxylic, cyclopentylcarboxylic, adamantoic, furic, nicotinic, thenoic, picolinic, benzoic, phenylacetic acid and the like.

Many of the 1-amino reactants (compounds of formula (IIIa) and (IIIb)) herein utilized for condensation with the 20-aldehyde function of rosaramicin or 12,13-desepoxy-12,13-dehydro-rosaramicin are commercially available. Those that must be synthesized may be prepared by one of the following general procedures.

A) J.H. Biel, *et.al., J. Org. Chem., 26* 4096 (1961)

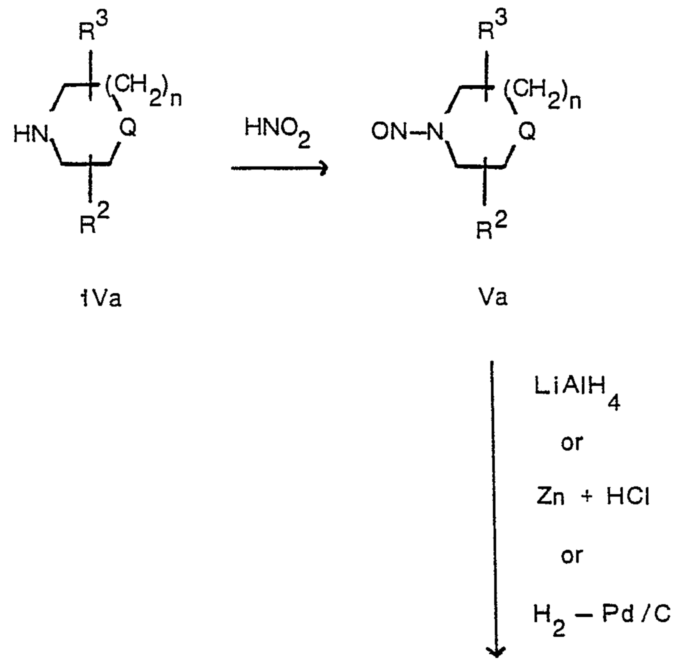


IVa

Va

IIIa

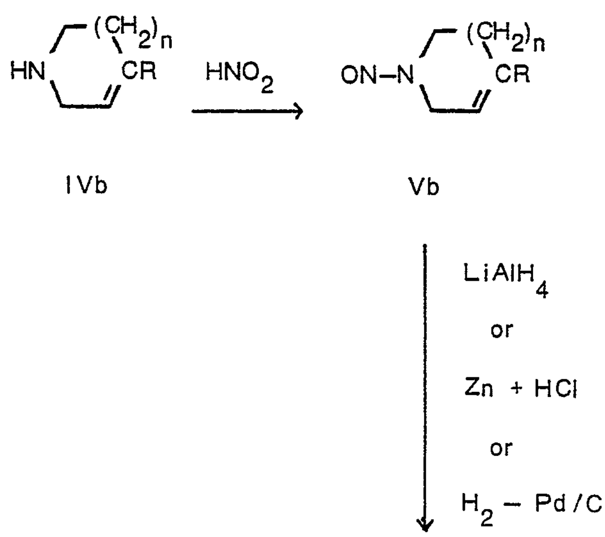


IVb

Vb

IIIb

for example:

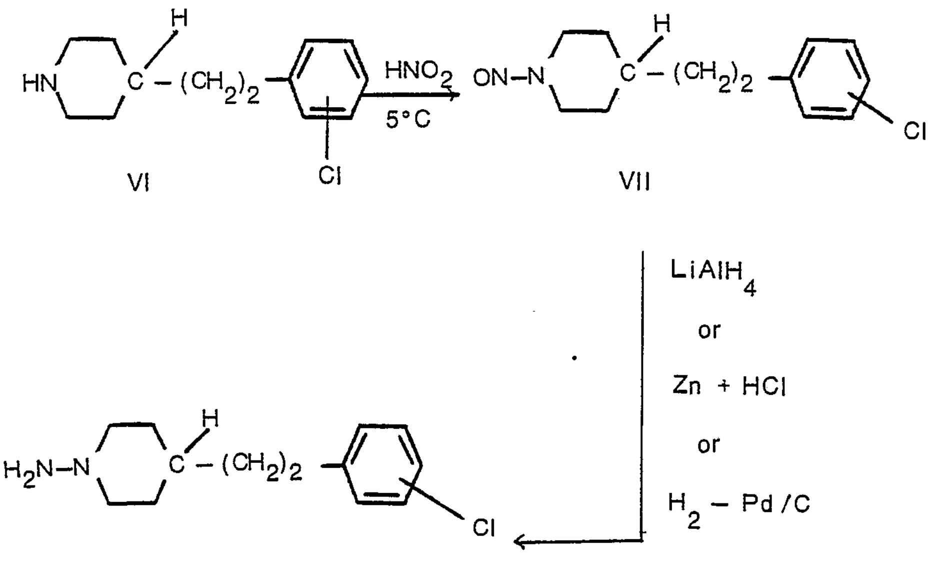


VI VII

VIII

B) R. Gosl *et.al., Org. Syn.,* Collec. Vol. V. 43, (1963).

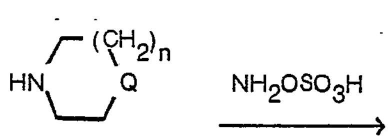


IIIa

IXa

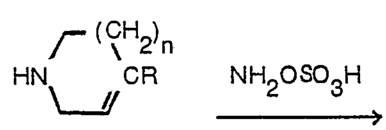


IIIb

IXb

for example:

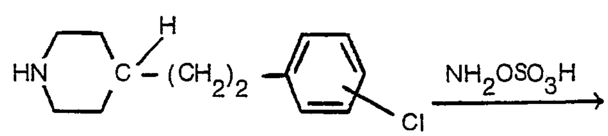


VIII

VI

Example 1

*20-[(1,1-Dioxothiomorpholino)imino]-20-deoxyrosaramicin*

Dissolve 1.09 g. of rosaramicin and 275 mg. of N-amino-1,1-dioxothiomorpholine in 50 ml of absolute ethanol. Stir the reaction mixture at room temperature for two days. Collect the white precipitate by filtration and crystallize from methanol. m.p. 180°C, $[\alpha]_D^{36°}$=–29.1; M=713.

Example 2

*20-[(4-Hydroxypiperidino)imino]-20-deoxyrosaramicin*

Dissolve 581 mg. of rosaramicin in 15 ml. of ethanol with stirring and add 120 mg. of 1-amino-4-hydroxypiperidine. Stir the reaction mixture at room temperature (20°C.) for 20 hours and evaporate. Dissolve the resulting yellow syrup in chloroform and chromatograph on a column containing 100 g. of silica gel. Develop the column using a solvent system consisting of 10% methanol in chloroform followed by 15% methanol in chloroform. Combine the fractions in accordance with the $R_f$'s on silica

gel plates using 5% methanol in chloroform to obtain thereby the product of this example, plus some unreacted rosaramicin.

In a similar manner, an equivalent quantity of the following 1-amino reactants can be subjected to the process of the foregoing examples to obtain thereby the corresponding substituted 20-imino-20-deoxy derivatives of rosaramicin and 12,13-desepoxy-12,13-dehydrorosaramicin, respectively:

N-amino-piperidine,
1-amino-4-methylpiperidine,
1-amino-4,4-ethylenedioxypiperidine,
1-amino-4-benzyloxypiperidine,
1-amino-4-methoxypiperidine,
1-amino-4-acetoxypiperidine,
1-amino-4-methyl-4-hydroxypiperidine,
1-amino-4-ethyl-4-propionyloxypiperidine,
1-amino-4-propyl-4-ethoxypiperidine,
1-amino-4-phenethylpiperidine,
1-amino-4-benzoyloxypiperidine,
1-amino-4-butoxycarbonylpiperidine,
1-amino-4-carboxypiperidine,
1-amino-4-dimethylaminocarbonylpiperidine,
1-amino-4-methylpiperazine,
1-amino-4-benzylpiperazine,
1-amino-4-phenethylpiperazine,
N-aminomorpholine,
N-amino-4-thiomorpholine,
1-amino-4-($\beta$-hydroxyethyl)piperazine,
1-amino-4-carbamoylpiperidine,
N-aminopyrrolidine,
N-aminohomopiperidine,
1-amino-2,6-dimethylpiperidine,
N-aminorhodanine,
1-aminohydantoin,
1-amino-4-phenylpiperidine,
1-amino-4-hydroxy-4-phenylpiperidine,
1-amino-4-cyano-4-phenylpiperidine,
1-amino-4-(p-chlorophenyl)-4-hydroxypiperidine,
1-amino-4-(p-chlorophenyl)-3,4-dehydropiperidine,
1-amino-4-(o-tolyl)piperazine,
1-amino-4-(m-tolyl)piperazine,
1-amino-4-($\alpha,\alpha,\alpha$-trifluoro-m-tolyl)piperazine,
1-amino-4-(benzyl)piperazine,
1-amino-4-(p-chlorobenzhydryl)piperazine,
1-amino-4-phenyl-3,4-dehydropiperidine,
1-amino-4-benzylpiperidine,
1-amino-4-phenylpiperazine,
1-amino-4-(p-fluorophenyl)piperazine,
1-amino-4-(o-chlorophenyl)piperazine,
1-amino-4-(m-chlorophenyl)piperazine,
1-amino-4-(p-chlorophenyl)piperazine,
1-amino-4-(o-methoxyphenyl)piperazine,
1-amino-4-(p-methoxyphenyl)piperazine,
1-amino-4-(p-acetylphenyl)piperazine.

The compounds of the general formula I can be exemplified by the following:

20-[(piperidino)imino]-20-deoxyrosaramicin,
20-[(4-methylpiperidino)imino]-20-deoxyrosaramicin,
20-[(4,4-ethylenedioxypiperidino)imino]-20-deoxyrosaramicin,
20-[(4-benzyloxypiperidino)imino]-20-deoxyrosaramicin,
20-[(4-methoxypiperidino)imino]-20-deoxyrosaramicin,
20-[(4-acetoxypiperidino)imino]-20-deoxyrosaramicin,
20-[(4-methyl-4-hydroxypiperidino)imino]-20-deoxyrosaramicin,
20-[(4-ethyl-4-propionyloxypiperidino)imino]-20-deoxyrosaramicin,
20-[(4-n-propyl-4-ethoxypiperidino)imino]-20-deoxyrosaramicin,
20-[(4-phenethylpiperidino)imino]-20-deoxyrosaramicin,

20-[(4-benzoyloxypiperidino)imino]-20-deoxyrosaramicin,
20-[(4-n-butoxycarbonylpiperidino)imino]-20-deoxyrosaramicin,
20-[(4-carboxypiperidino)imino]-20-deoxyrosaramicin,
20-[(4-dimethylaminocarbonylpiperidino)imino]-20-deoxyrosaramicin,
20-[(4-methyl-1-piperazinyl)imino]-20-deoxyrosaramicin,
20-[(4-benzyl-1-piperazinyl)imino]-20-deoxyrosaramicin,
20-[(4-phenethyl-1-piperazinyl)imino]-20-deoxyrosaramicin,
20-[(morpholino)imino]-20-deoxyrosaramicin,
20-[(thiomorpholino)imino]-20-deoxyrosaramicin,
20-[(4-β-hydroxyethyl-1-piperazinyl)imino]-20-deoxyrosaramicin,
20-[(4-carbamoylpiperidino)imino]-20-deoxyrosaramicin,
20-[(1-pyrrolidinyl)imino]-20-deoxyrosaramicin,
20-[(1-homopiperidyl)imino]-20-deoxyrosaramicin,
20-[(2,6-dimethylpiperidino)imino]-20-deoxyrosaramicin,
20-[(4-oxo-2-thioxo-3-thiazolidinyl)imino]-20-deoxyrosaramicin,
20-[(2,4-dioxo-1-imidazolidinyl)imino]-20-deoxyrosaramicin,
20-[(4-phenylpiperidino)imino]-20-deoxyrosaramicin,
20-[(4-hydroxy-4-phenylpiperidino)imino]-20-deoxyrosaramicin,
20-[(4-cyano-4-phenylpiperidino)imino]-20-deoxyrosaramicin,
20-{[4-(p-chlorophenyl)-4-hydroxypiperidino]imino)}-20-deoxyrosaramicin,
20-{[4-(p-chlorophenyl)-3,4-dehydropiperidino]-imino}-20-deoxyrosaramicin,
20-{[4-(o-tolyl)-1-piperazinyl]imino}-20-deoxyrosaramicin,
20-{[m-tolyl)-1-piperazinyl]imino}-20-deoxyrosaramicin,
20-{[4-(α,α,α-trifluoro-m-tolyl)-1-piperazinyl]imino}-20-deoxyrosaramicin,
20-[(4-benzyl-1-piperazinyl)imino]-20-deoxyrosaramicin,
20-{[4-(p-chlorobenzhydryl)-1-piperazinyl]-imino}-20-deoxyrosaramicin,
20-[(4-phenyl-3,4-dehydropiperidino)imino]-20-deoxyrosaramicin,
20-[(4-benzylpiperidino)imino]-20-deoxyrosaramicin,
20-[(4-phenyl-1-piperazinyl)imino]-20-deoxyrosaramicin,
20-{[4-(p-fluorophenyl)-1-piperazinyl]imino}-20-deoxyrosaramicin,
20-{[4-(o-chlorophenyl)-1-piperazinyl]imino}-20-deoxyrosaramicin,
20-{[4-(m-chlorophenyl)-1-piperazinyl]amino}-20-deoxyrosaramicin,
20-{[4-(p-chlorophenyl)-1-piperazinyl]imino}-20-deoxyrosaramicin,
20-{[4-(o-methoxyphenyl)-1-piperazinyl]imino}-20-deoxyrosaramicin,
20-[4-(p-methoxyphenyl)-1-piperazinyl]imino)-20-deoxyrosaramicin,
20-{[4-(p-acetylphenyl)-1-piperazinyl]imino}-20-deoxyrosaramicin,
20-[(1-piperazinyl)imino]-20-deoxyrosaramicin,
20-[(4-ethyl-4-hydroxypiperidino)imino]-20-deoxyrosaramicin,
20-[(4-ethyl-4-methoxypiperidino)imino]-20-deoxyrosaramicin,
20-[(4-methyl-4-propionyloxypiperidino)imino]-20-deoxyrosaramicin,
20-[(4-methoxycarbonylpiperidino)imino]-20-deoxyrosaramicin,
12,13-desepoxy-12,13-dehydro-20-[(piperidino]imino]-20-deoxyrosaramicin,
12,13-desepoxy-12,13-dehydro-20-[(1-piperazinyl)imino]-20-deoxyrosaramicin,
12,13-desepoxy-12,13-dehydro-20-[(4-methyl-1-piperazinyl)imino]-20-deoxyrosaramicin,
12,13-desepoxy-12,13-dehydro-20-[(morpholino)imino]-20-deoxyrosaramicin,
12,13-desepoxy-12,13-dehydro-20-[(thiomorpholino)imino]-20-deoxyrosaramicin,
12,13-desepoxy-12,13-dehydro-20-[(1,1-dioxothiomorpholino)imino]-20-deoxyrosaramicin,
12,13-desepoxy-12,13-dehydro-20-[(4,4-ethylenedioxypiperidino)imino]-20-deoxyrosaramicin,
12,13-desepoxy-12,13-dehydro-20-[(4-hydroxypiperidino)imino]-20-deoxyrosaramicin,
12,13-desepoxy-12,13-dehydro-20-[(4-ethoxypiperidino)imino]-20-deoxyrosaramicin,
12,13-desepoxy-12,13-dehydro-20-[(4-benzoyloxypiperidino)imino]-20-deoxyrosaramicin,
12,13-desepoxy-12,13-dehydro-20-[(4-propyl-4-hydroxypiperidino)imino]-20-deoxyrosaramicin,
12,13-desepoxy-12,13-dehydro-20-[(4-methyl-4-n-butoxypiperidino)imino]-20-deoxyrosaramicin,
12,13-desepoxy-12,13-dehydro-20-[(4-pentyl-4-propionyloxypiperidino)imino]-20-deoxyrosaramicin,
12,13-desepoxy-12,13-dehydro-20-[(4-carboxypiperidino)imino]-20-deoxyrosaramicin,
12,13-desepoxy-12,13-dehydro-20-[(4-methoxycarbonylpiperidino)imino]-20-deoxyrosaramicin,
12,13-desepoxy-12,13-dehydro-20-[(4-carbamoylpiperidino)imino]-20-deoxyrosaramicin, and
12,13-desepoxy-12,13-dehydro-20-[(dimethylaminocarbonylpiperidino)imino]-20-deoxyrosaramicin.

Selected macrolides of this invention were subjected to art recognized tests to determine their antibacterial profile.

In vitro broth dilution tests (MIC's) were done in conventional manner using Mueller-Hinton broth at a pH of 7,2, (Table I) and 7,4 (Table II), in volumes of 3—5 ml/tube. Inocula were obtained by diluting overnight cultures. Tubes were incubated at 37°C and endpoints were read after 24 and 48 hours.

In the following tables MIC's (minimum inhibitory concentrations) of the following compounds are listed:

A: 20-[(1,1-Dioxothiomorpholino)imino]-20-deoxyrosaramicin,
B: 20-[(4-methyl-1-piperazinyl)imino]-20-deoxyrosaramicin,
C: 12,13-desepoxy-12,13-dehydro-20-[(4,4-ethylenedioxypiperidino)-imino]-20-deoxyrosaramicin,
D: 20-[(4-Hydroxypiperidino)imino]-20-deoxyrosaramicin,
E: 12,13-desepoxy-12,13-dehydro-20-[(thiomorpholino)imino]-20-deoxyrosaramicin,
F: 12,13-desepoxy-12,13-dehydro-20-[(1,1-dioxothiomorpholino)-imino]-20-deoxyrosaramicin.

TABLE I
MIC's [mcg/ml] of compound F

| ORGANISM | 24 HOURS | 48 HOURS |
|---|---|---|
| B. Subtilis 6633 | 0.125 | 0.125 |
| E. Coli JR66 Ant-(2') | 8.00 | 8.00 |
| „ „ 589 | 8.00 | 8.00 |
| Enterobacter 72012503 | 32.00 | 32.00 |
| Klebsiella pneumoniae Adler (7) | 32.00 | 32.00 |
| " " GT3020 | 4.00 | 4.00 |
| Providencia 164 AAC-(2') | 64.00 | 64.00 |
| " 72052305 AAC-(2') | 64.00 | 64.00 |
| Pseudomonas Aeruginosa — 3223 | 64.00 | 64.00 |
| " " — 75022103-(2') | 64.00 | 64.00 |
| Salmonella 77062009 | 8.00 | 8.00 |
| Sarcina lutea 9341 | 0.625 | 0.625 |
| Serratia Marcescens 75082819 | 16.00 | 16.00 |
| Shigella dysenteriae 13313 | 2.00 | 4.00 |
| Staphylococcus Gray | 0.125 | 0.125 |
| „ Wood | 0.125 | 0.125 |
| Streptococcus agalactiae Gionti Group B | 1.00 | 1.00 |
| Streptococcus Thacker Group C | 0.125 | 0.250 |
| Streptococcus Parsons Group G | 0.50 | 0.50 |
| Streptococcus pneumoniae VA 62 | 0.250 | 0.250 |
| Streptococcus pyogenes Gionti Group A | 0.500 | 0.500 |
| Streptococcus Viridans 1141 | 0.500 | 0.500 |

TABLE II
MIC's [mcg/ml] OF SELECTED MACROLIDES/Mueller Hinton Broth

| Organism | | A | | B | | C | | D | | E | |
|---|---|---|---|---|---|---|---|---|---|---|---|
| | | 24 hr | 48 hr | 24 hr | 48 hr | 24 hr | 48 hr | 24 hr | 48 hr | 24 hr | 48 hr |
| *Staph.* aureus | 209P | 0.03 | .25 | .125 | .25 | .125 | .5 | .06 | .25 | .125 | .25 |
| " | Wood | <0.015 | .06 | .125 | .25 | .125 | .25 | .125 | .25 | .125 | .25 |
| " | Ziegler | .03 | .25 | .125 | .25 | .25 | .25 | .125 | .25 | .25 | .5 |
| " | 59N | .03 | .06 | .06 | .25 | .125 | .5 | .125 | .5 | .125 | .25 |
| " | 1613 | .03 | .125 | .125 | .25 | .06 | .25 | .125 | .25 | .25 | .5 |
| " | 168 | <.015 | .06 | .125 | .25 | .125 | .25 | .125 | .25 | .125 | 1 |
| " | 676 | .03 | .125 | .125 | .25 | .125 | .5 | .125 | .5 | .125 | .5 |
| " | 723N | .03 | .125 | .125 | .25 | .125 | .25 | .125 | .5 | .125 | .25 |
| " | 572 | .06 | .06 | .06 | .25 | .06 | .25 | .06 | .25 | .125 | .25 |
| " | 306 | .03 | .06 | .25 | .5 | .125 | .5 | .25 | .5 | .5 | 1 |
| *Strep.* | Karipedes | .125 | .25 | .03 | .125 | | | | | .03 | .125 |
| " | Alvarez | 0.5 | .5 | .25 | .5 | | | | | .5 | 1 |
| " | C | .125 | .25 | .125 | .25 | 8 | 16 | 8 | 16 | .25 | .5 |
| " | Cruz | .125 | .25 | .25 | .5 | .5 | 2 | 1 | 1 | .125 | .5 |

TABLE II (continued)
MIC's [mcg/ml] OF SELECTED MACROLIDES/Mueller Hinton Broth

| Organism | | A 24 hr | A 48 hr | B 24 hr | B 48 hr | C 24 hr | C 48 hr | D 24 hr | D 48 hr | E 24 hr | E 48 hr |
|---|---|---|---|---|---|---|---|---|---|---|---|
| *Enterococcus* | 1022 | .03 | .25 | .125 | .25 | .06 | .25 | .06 | .25 | .125 | .5 |
| " | 1053 | >16 | — | >32 | — | >16 | — | >16 | — | 32 | >32 |
| " | 373 | <.015 | .125 | .125 | .25 | .06 | .25 | .06 | .125 | .25 | .5 |
| " | 0969/72 | <.015 | .125 | .125 | .25 | .125 | .5 | .25 | .25 | .125 | .5 |
| *E. Coli* | 15741 | 1 | 2 | 2 | 4 | 4 | 8 | 2 | 4 | 2 | 4 |
| " | Baker 2 | >16 | — | >32 | — | 16 | >16 | >16 | — | 32 | >32 |
| " | ATCC 10536 | .25 | .25 | 1 | 2 | 1 | 4 | 1 | 2 | 2 | 4 |
| " | Behrens | 1 | 1 | 2 | 4 | 2 | 4 | 4 | 4 | 2 | 4 |
| " | Jordan 1 | 1 | 1 | 8 | 16 | 4 | 8 | 4 | 8 | 8 | 16 |
| *Kleb.* | 3020 | | <.015 | 8 | 8 | .125 | 2 | .25 | 4 | 4 | 8 |
| " | Rahal 3 | 4 | 4 | 8 | 16 | 4 | 8 | 2 | 8 | 8 | 8 |
| " | Brooke Meads | >16 | — | 32 | 32 | >16 | — | >16 | — | 32 | 32 |
| *Ps.* aerug. | Cohen McCl | 16 | >16 | 16 | 16 | 8 | >16 | 16 | 16 | 8 | 16 |
| " | " Wrig | >16 | — | 8 | 16 | >16 | — | 16 | >16 | 8 | 16 |
| " | Rahal 324 | 8 | 16 | 8 | 16 | 8 | 16 | 16 | >16 | 8 | 16 |

TABLE II (continued)
MIC's [mcg/ml] OF SELECTED MACROLIDES/Mueller Hinton Broth

| Organism | | A | | B | | C | | D | | E | |
|---|---|---|---|---|---|---|---|---|---|---|---|
| | | 24 hr | 48 hr | 24 hr | 48 hr | 24 hr | 48 hr | 24 hr | 48 hr | 24 hr | 48 hr |
| *Serratia* | Brooke Marces | 8 | 16 | 8 | 16 | 16 | 16 | 16 | 16 | 8 | 8 |
| " | Dalton | 8 | 16 | 8 | 16 | 16 | 16 | 16 | >16 | 4 | 8 |
| *Salm.* Gr. B Typhim | | 2 | 4 | 8 | 16 | 8 | 16 | 8 | 16 | 8 | 16 |
| *Proteus* | *Mirab.* Harding | 16 | >16 | 32 | 32 | 16 | 16 | >16 | — | 8 | 16 |
| *Proteus* | *Rett* Anderson | 16 | >16 | 32 | 32 | >16 | — | >16 | — | 16 | 32 |
| B. Subt. | ATCC 6633 | .03 | .06 | .06 | .25 | .25 | .05 | .125 | .25 | .125 | .25 |
| C. alb. | Wisc. | >16 | — | 8 | >32 | 8 | >16 | 4 | >16 | 4 | >32 |
| *Staph.* | Gray | 0.125 | .25 | | | .25 | .5 | .25 | .25 | | |
| " | Giorgio | <.015 | .06 | | | .125 | .25 | .125 | .25 | | |
| " | AF1 | .03 | .06 | | | .125 | .25 | .125 | .25 | | |
| " | 832 | .03 | .125 | | | .25 | .5 | .5 | .25 | | |
| " | 892 | .03 | .03 | | | .25 | .5 | .25 | .25 | | |
| " | 887 | .03 | .03 | | | .06 | .25 | .125 | .125 | | |

TABLE III

$PD_{50}$ (against Staphylococcus aureus) of selected macrolides [mg/kg]

| compound | B | E |
|---|---|---|
| s.c.: | 5 | 2,5 |
| oral: | 50 | 25 |

12,13-desepoxy-12,13-dehydro-20-[(1,1-dioxothiomorpholino)imino]-20-deoxyrosaramicin, m.p. 157°—160°C

$[\alpha]_D^{26°} = -57.1°$

NMR (solvent: acetone $- d_6$)

| | |
|---|---|
| $CH_3$ | 0.93 triplet (j=7.0Hz) |
| $CH_3$ | 1.09 doublet (j=6.5) ×2 |
| | 1.21 doublet (j=6.5) ×2 |
| | 1.22 doublet (j=6.5) ×2 |
| $CH_3$ | 1.88 doublet (j=1.5Hz) |
| $N(CH_3)_2$ | 2.29 singlet |
| 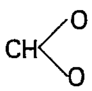 | 4.25 doublet (j=8.0Hz, [Desosamine]) |
| 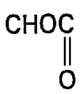 | 4.70 multiplet (j=9.0, 9.0, 2.5) |
| =CH | 5.69 doublet (j=10.0Hz), broad also coupled to $CH_3$ group |
| =CH | 6.59 doublet (j=16.0Hz) |
| =CH | 7.27 doublet (j=16.0Hz) |
| HC=N | 7.15 triplet (j=5.0, 5.0Hz) |

12,13-desepoxy-12,13-dehydro-20-[(4-ethylenedioxypiperidino)- imino]-20-deoxyrosaramicin
NMR (solvent: $(CD_3)_2$ CO):

| | |
|---|---|
| $CH_3$ | 0.92, t (j=7.0Hz) |
| $CH_3$ | 1.08, 1.10, 1.22, 1.23 doublets (j=6.5Hz) |
| $CH_3$ | 1.88, d, j=1.5Hz |
| $N(CH_3)_2$ | 2.28, singlet |
| $O—CH_2—CH_2—O$ | 3.92, singlet |
| 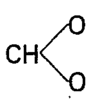 | 4.22, doublet j=8.0 |
| 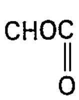 | 4.68, multiplet (9.0, 9.0, 2.5) |

TABLE III (continued)

| | |
|---|---|
| =CH | 5.65, doublet (j=10.0) broad due to long-range coupling to $CH_3$ group |
| =CH | 6.52, doublet, 16.0Hz |
| =CH | 7.20, doublet, 16.0Hz |
| HC=N | 6.98, triplet j=5.0, 5.0Hz |
| 20-[(4-Hydroxypiperidino)imino]-20-deoxyrosaramicin, NMR (solvent: $(CD_3)_2$ CO): | |
| $CH_3$ | 0.90, t (j=7.0Hz) |
| $CH_3$ | 1.05, 1.10, 1.20, 1.18 doublets (j=6.5Hz) |
| $CH_3$ | 1.48, singlet |
| $N(CH_3)_2$ | 2.26, singlet |
| 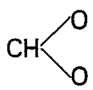 | 4.25, doublet, j=8.0Hz |
| 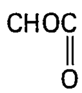 | 4.82 multiplet |
| =CH | 6.37, doublet, j=16.0Hz |
| =CH | 6.80 doublet, j=16.0Hz |
| HC=N | 6.95 triplet, j=5.0, 5.0Hz |

**Claims**

1. A compound of the general formula

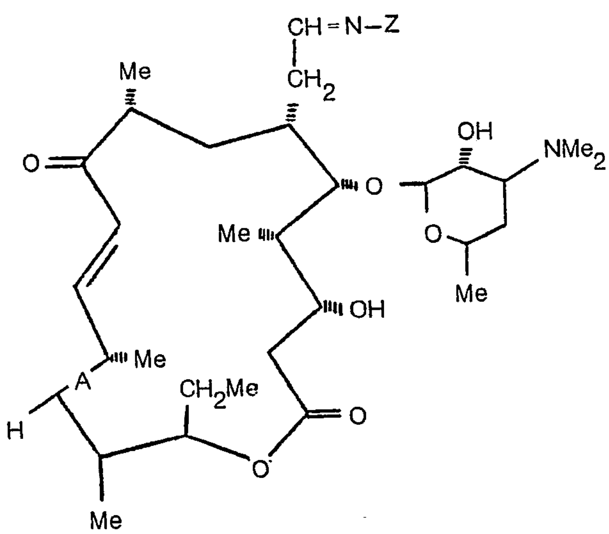

I

wherein Me is methyl;
A represents a double bond between the carbon atoms of positions 12 and 13 or, together with the carbon atoms of positions 12 and 13, an oxirane ring;
Z is a group of formula IIa or IIb,

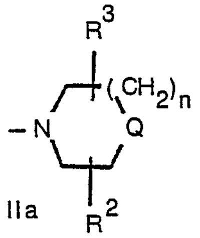

IIa

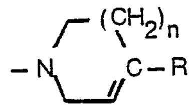

IIb

wherein Q is $CRR^1$, NR, O, S, $SO_2$,

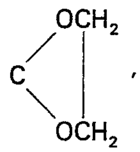,

$CROR^1$, $CROC(O)R^1$, CHCOOR, $CHCONRR^1$ or CRCN; wherein R and $R^1$ independent of each other represent hydrogen, alkyl containing 1 to 8 carbon atoms, aralkyl containing 7 to 13 carbon atoms or aryl containing 6 to 10 carbon atoms, the aryl groups being unsubstituted or being substituted by halogen, trifluoromethyl, alkoxy containing 1 to 5 carbon atoms or alkylcarbonyl containing 1 to 5 carbon atoms or alkyl containing 1 to 5 carbon atoms and when one of R and $R^1$ is hydrogen the other one can also be hydroxyalkyl containing 1 to 8 carbon atoms; $R^2$ and $R^3$ independent of each other are hydrogen, alkyl containing 1 to 8 carbon atoms, oxo or thioxo; n is zero, 1 or 2;
or a pharmaceutically acceptable acid addition salt thereof.

2. A compound of formula I according to claim 1, wherein Z is a group of formula (IIa), wherein $R^2$ and $R^3$ are hydrogen and Me, A, Q and n are defined as in claim 1.

3. A compound of formula I according to claim 2, wherein Q is $CRR^1$, NR, O, S, $SO_2$,

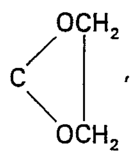,

$CROR^1$, $CROC(O)R^1$, CHCOOR, $CHCONRR^1$ or CRCN; wherein R and $R^1$ independent of each other represent hydrogen, alkyl containing 1 to 5 carbon atoms, aralkyl containing 7 to 13 carbon atoms or aryl containing 6 carbon atoms, the aryl groups being unsubstituted or being substituted by fluoro, chloro, trifluoromethyl, methoxy, acetyl and methyl, and when one of R and $R^1$ is hydrogen the other one can also be hydroxyalkyl containing 1 or 2 carbon atoms.

4. A compound of formula I according to claim 3, wherein n is zero or one.

5. A compound of formula I according to claim 3, wherein Me and A are defined as in claim 1, Z is a group of formula (IIa), wherein $R^2$ and $R^3$ are hydrogen, Q is $NCH_3$, S, $SO_2$,

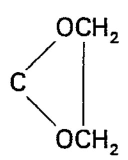

or CHOH and n is one.

6. A compound of formula I according to claim 3, which is
20-[(1,1-Dioxothiomorpholino)imino]-20-deoxyrosaramicin,
20-[(4-Hydroxypiperidino)imino]-20-deoxyrosaramicin,
20-[(piperidino)imino]-20-deoxyrosaramicin,
20-[(4,4-ethylenedioxypiperidino)imino]-20-deoxyrosaramicin,
20-[(4-methoxypiperidino)imino]-20-deoxyrosaramicin,
20-[(4-acetoxypiperidino)imino]-20-deoxyrosaramicin,
20-[(4-carboxypiperidino)imino]-20-deoxyrosaramicin,
20-[(4-dimethylaminocarbonylpiperidino)imino]-20-deoxyrosaramicin,
20-[(4-methyl-1-piperazinyl)imino]-20-deoxyrosaramicin,
20-[(morpholino)imino]-20-deoxyrosaramicin,
20-[(thiomorpholino)imino]-20-deoxyrosaramicin,
20-[(4-carbamoylpiperidino)imino]-20-deoxyrosaramicin,

20-[(1-pyrrolidinyl)imino]-20-deoxyrosaramicin,
20-[(1-homopiperidyl)imino]-20-deoxyrosaramicin,
20-[(1-piperazinyl)imino]-20-deoxyrosaramicin,
12,13-desepoxy-12,13-dehydro-20-[(1-piperazinyl)imino]-20-deoxyrosaramicin,
12,13-desepoxy-12,13-dehydro-20-[(4-methyl-1-piperazinyl)imino]-20-deoxyrosaramicin,
12,13-desepoxy-12,13-dehydro-20-[(morpholino)imino]-20-deoxyrosaramicin,
12,13-desepoxy-12,13-dehydro-20-[(thiomorpholino)imino]-20-deoxyrosaramicin,
12,13-desepoxy-12,13-dehydro-20-[(1,1-dioxothiomorpholino)imino]-20-deoxyrosaramicin,
12,13-desepoxy-12,13-dehydro-20-[(4,4-ethylenedioxypiperidino)imino]-20-deoxyrosaramicin,
12,13-desepoxy-12,13-dehydro-20-[(4-hydroxypiperidino)imino]-20-deoxyrosaramicin,
12,13-desepoxy-12,13-dehydro-20-[(4-ethoxypiperidino)imino]-20-deoxyrosaramicin,
12,13-desepoxy-12,13-dehydro-20-[(4-benzoyloxypiperidino)imino]-20-deoxyrosaramicin,
12,13-desepoxy-12,13-dehydro-20-[(4-propyl-4-hydroxypiperidino)imino]-20-deoxyrosaramicin,
12,13-desepoxy-12,13-dehydro-20-[(4-methyl-4-n-butoxypiperidino)imino]-20-deoxyrosaramicin,
12,13-desepoxy-12,13-dehydro-20-[(4-pentyl-4-propionyloxypiperidino)imino]-20-deoxyrosaramicin,
12,13-desepoxy-12,13-dehydro-20-[(4-carboxypiperidino)imino]-20-deoxyrosaramicin,
20-[(4-ethyl-4-hydroxypiperidino)imino]-20-deoxyrosaramicin,
20-[(4-ethyl-4-methoxypiperidino)imino]-20-deoxyrosaramicin,
20-[(4-methyl-4-propionyloxypiperidino)imino]-20-deoxyrosaramicin,
20-[(4-methoxycarbonylpiperidino)imino]-20-deoxyrosaramicin,
12,13-desepoxy-12,13-dehydro-20-[(piperidino)imino]-20-deoxyrosaramicin,
12,13-desepoxy-12,13-dehydro-20-[(4-methoxycarbonylpiperidino)imino]-20-deoxyrosaramicin,
12,13-desepoxy-12,13-dehydro-20-[(4-carbamoylpiperidino)imino]-20-deoxyrosaramicin, and
12,13-desepoxy-12,13-dehydro-20-[(dimethylaminocarbonylpiperidino)imino]-20-deoxyrosaramicin.

7. A compound of formula I according to claim 1, wherein Z is a group of formula (IIa), Q is $CH_2$, $R^2$ and $R^3$ independent of each other are alkyl containing 1 to 8 carbon atoms, oxo or thioxo, n is zero or one and Me and A are defined as in claim 1.

8. A compound according to claim 7, which is
20-[(2,6-dimethylpiperidino)imino]-20-deoxyrosaramicin,
20-[(4-oxo-2-thioxo-3-thiazolidinyl)imino]-20-deoxyrosaramicin, or
20-[(2,4-dioxo-1-imidazolidinyl)imino]-20-deoxyrosaramicin.

9. A compound of formula I according to claim 1, wherein Z is a group of formula (IIb), n is one and Me, A and R are as defined in claim 1.

10. A compound according to claim 9, which is
20-{[4-(p-chlorophenyl)-3,4-dehydropiperidino]-imino}-20-deoxyrosaramicin or
20-[(4-phenyl-3,4-dehydropiperidino)imino]-20-deoxyrosaramicin.

11. Process for the preparation of compounds of the general formula I set forth in claim 1 and the pharmaceutically acceptable acid addition salts thereof, characterized in that rosaramicin or 12,13-desepoxy-12,13-dehydrorosaramicin is reacted with a compound of the general formula

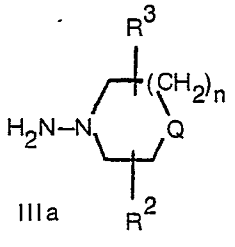

IIIa

or

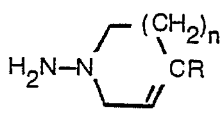

IIIb

wherein n, Q, R, $R^2$ and $R^3$ are defined as in any one of claims 1 to 10 and the so obtained compound is isolated in the free form or in the form of its pharmaceutically acceptable acid addition salts.

12. A compound of the general formula (I) as defined in any one of claims 1 to 10 or obtained by the process of claim 11 for use in treating infections.

13. A pharmaceutical composition comprising as active ingredient at least one compound of the general formula (I) as defined in any one of claims 1 to 10 or obtained by a process of claim 11 together with a pharmaceutically acceptable carrier.

**Patentansprüche**

1. Verbindung der allgemeinen Formel

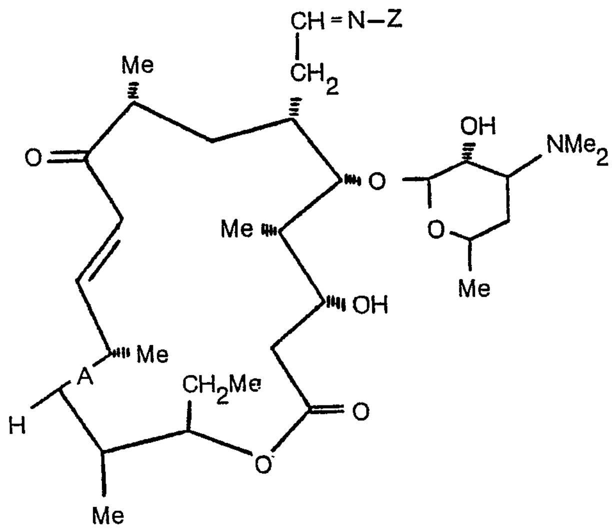

in der
Me Methyl ist,
A eine Doppelbindung zwischen den Kohlenstoff-Atomen 12 und 13 oder, zusammen mit den Kohlenstoff-Atomen in den Stellungen 12 und 13, einen Oxiran-Ring bezeichnet,
Z eine Gruppe der Formel IIa oder IIb ist

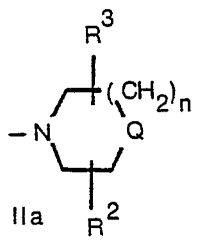

IIa

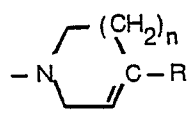

II b

in der
Q $CRR^1$, NR, O, S, $SO_2$,

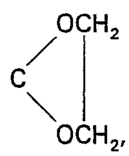

,

$CROR^1$, $CROC(O)R^1$, CHCOOR, $CHCONRR^1$ oder CRCN ist, worin
R und $R^1$ unabhängig voneinander Wasserstoff, Alkyl mit 1 bis 8 Kohlenstoff-Atomen, Aralkyl mit 7 bis 13 Kohlenstoff-Atomen oder Aryl mit 6 bis 10 Kohlenstoff-Atomen bezeichnen, wobei die Aryl-Gruppen unsubstituiert oder durch Halogen, Trifluoromethyl, Alkoxy mit 1 bis 5 Kohlenstoff-Atomen oder Alkylcarbonyl mit 1 bis 5 Kohlenstoff-Atomen oder Alkyl mit 1 bis 5 Kohlenstoff-Atomen substituiert sind, und in dem Fall, in dem einer der Substituenten R und $R^1$ Wasserstoff ist, der andere auch Hydroxyalkyl mit 1 bis 8 Kohlenstoff-Atomen sein kann,
$R^2$ und $R^3$ unabhängig voneinander Wasserstoff, Alkyl mit 1 bis 8 Kohlenstoff-Atomen, Oxo oder Thioxo ist und
n null, 1 oder 2 ist,
oder ein pharmazeutisch unbedenkliches Säureadditions-salz derselben.

2. Verbindung der Formel I nach Anspruch 1, dadurch gekennzeichnet, daß Z eine Gruppe der Formel (IIa) ist, in der $R^2$ und $R^3$ Wasserstoff sind und Me, A, Q und n die in Anspruch 1 angegebene Bedeutung haben.

3. Verbindung der Formel I nach Anspruch 2, dadurch gekennzeichnet, daß
Q $CRR^1$, NR, O, S, $SO_2$,

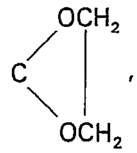

$CROR^1$, $CROC(O)R^1$, $CHCOOR$, $CHCONRR^1$ oder $CRCN$ ist, worin R und $R^1$ unabhängig voneinander Wasserstoff, Alkyl mit 1 bis 5 Kohlenstoff-Atomen, Aralkyl mit 7 bis 13 Kohlenstoff-Atomen oder Aryl mit 6 Kohlenstoff-Atomen bezeichnen, wobei die Aryl-Gruppen unsubstituiert oder durch Fluoro, Chloro, Trifluoromethyl, Methoxy, Acetyl und Methyl substituiert sind, und in dem Fall, in dem einer der Substituenten R und $R^1$ Wasserstoff ist, der andere auch Hydroxyalkyl mit 1 bis 2 Kohlenstoff-Atomen sein kann.

4. Verbindung der Formel I nach Anspruch 3, dadurch gekennzeichnet, daß n null oder eins ist.

5. Verbindung der Formel I nach Anspruch 3, dadurch gekennzeichnet, daß Me und A die in Anspruch 1 angegebene Bedeutung haben, Z eine Gruppe der Formel (IIa) ist, $R^2$ und $R^3$ Wasserstoff sind, Q $NCH_3$, S, $SO_2$,

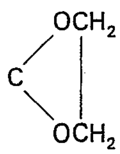

oder CHOH ist und n eins ist.

6. Verbindung der Formel I nach Anspruch 3
20-[(1,1-Dioxothiomorpholino)imino]-20-desoxyrosaramicin,
20-[(4-Hydroxypiperidino)imino]-20-desoxyrosaramicin,
20-[(Piperidino)imino]-20-desoxyrosaramicin,
20-[(4,4-Ethylendioxypiperidino)imino]-20-desoxyrosaramicin,
20-[(4-Methoxypiperidino)imino]-20-desoxyrosaramicin,
20-[(4-Acetoxypiperidino)imino]-20-desoxyrosaramicin,
20-[(4-Carboxypiperidino)imino]-20-desoxyrosaramicin,
20-[(4-Dimethylaminocarbonylpiperidino)imino]-20-desoxyrosaramicin,
20-[(4-Methyl-1-piperazinyl)imino]-20-desoxyrosaramicin,
20-[(Morpholino)imino]-20-desoxyrosaramicin,
20-[(Thiomorpholino)imino]-20-desoxyrosaramicin,
20-[(4-Carbamoylpiperidino)imino]-20-desoxyrosaramicin,
20-[(1-Pyrrolidinyl)imino]-20-desoxyrosaramicin,
20-[(1-Homopiperidyl)imino]-20-desoxyrosaramicin,
20-[(1-Piperazinyl)imino]-20-desoxyrosaramicin,
12,13-Desepoxy-12,13-dehydro-20-[(1-piperazinyl)imino]-20-desoxyrosaramicin,
12,13-Desepoxy-12,13-dehydro-20-[(4-methyl-1-piperazinyl)imino]-20-desoxyrosaramicin,
12,13-Desepoxy-12,13-dehydro-20-[(morpholino)imino]-20-desoxyrosaramicin,
12,13-Desepoxy-12,13-dehydro-20-[(thiomorpholino)imino]-20-desoxyrosaramicin,
12,13-Desepoxy-12,13-dehydro-20-[(1,1-dioxothiomorpholino)imino]-20-desoxyrosaramicin,
12,13-Desepoxy-12,13-dehydro-20-[(4,4-ethylendioxypiperidino)imino]-20-desoxyrosaramicin,
12,13-Desepoxy-12,13-dehydro-20-[(4-hydroxypiperidino)imino]-20-desoxyrosaramicin,
12,13-Desepoxy-12,13-dehydro-20-[(4-ethoxypiperidino)imino]-20-desoxyrosaramicin,
12,13-Desepoxy-12,13-dehydro-20-[(4-benzoyloxypiperidino)imino]-20-desoxyrosaramicin,
12,13-Desepoxy-12,13-dehydro-20-[(4-propyl-4-hydroxypiperidino)imino]-20-desoxyrosaramicin,
12,13-Desepoxy-12,13-dehydro-20-[(4-methyl-4-n-butoxypiperidino)imino]-20-desoxyrosaramicin,
12,13-Desepoxy-12,13-dehydro-20-[(4-pentyl-4-propionyloxypiperidino)imino]-20-desoxyrosaramicin,
12,13-Desepoxy-12,13-dehydro-20-[(4-Carboxypiperidino)imino]-20-desoxyrosaramicin,
20-[(4-Ethyl-4-hydroxypiperidino)imino]-20-desoxyrosaramicin,
20-[(4-Ethyl-4-methoxypiperidino)imino]-20-desoxyrosaramicin,
20-[(4-Methyl-4-propionyloxypiperidino)imino]-20-desoxyrosaramicin,
20-[(4-Methoxycarbonylpiperidino)imino]-20-desoxyrosaramicin,
12,13-Desepoxy-12,13-dehydro-20-[(piperidino)imino]-20-desoxyrosaramicin,
12,13-Desepoxy-12,13-dehydro-20-[(4-methoxycarbonylpiperidino)imino]-20-desoxyrosaramicin,
12,13-Desepoxy-12,13-dehydro-20-[(4-carbamoylpiperidino)imino]-20-desoxyrosaramicin und
12,13-Desepoxy-12,13-dehydro-20-[(4-dimethylaminocarbonylpiperidino)imino]-20-desoxyrosaramicin,

7. Verbindung der Formel I nach Anspruch 1, dadurch gekennzeichnet, daß Z eine Gruppe der Formel (IIa) ist, Q $CH_2$ ist, $R^2$ und $R^3$ unabhängig voneinander Alkyl mit 1 bis 8 Kohlenstoff-Atomen, Oxo

oder Thioxo sind, n null oders eins ist und Me und A die in Anspruch 1 angegebene Bedeutung haben.

8. Verbindung nach Anspruch 7
20-[(2,6-Dimethylpiperidino)imino]-20-desoxyrosaramicin,
20-[(4-Oxo-2-thioxo-3-thiazolidinyl)imino]-20-desoxyrosaramicin oder
20-[(2,4-Dioxo-1-imidazolidinyl)imino]-20-desoxyrosaramicin.

9. Verbindung der Formel I nach Anspruch 1, dadurch gekennzeichnet, daß Z eine Gruppe der Formel (IIb) ist, n eins ist und Me, A und R die in Anspruch 1 angegebene Bedeutung haben.

10. Verbindung nach Anspruch 9
20-{[4(p-Chlorophenyl)-3,4-dehydropiperidino]imino}-20-desoxyrosaramicin oder
20-[(4-Phenyl-3,4-dehydropiperidino)imino]-20-desoxyrosaramicin.

11. Verfahren zur Herstellung von Verbindungen der in Anspruch 1 bezeichneten allgemeinen Formel I und der pharmazeutisch unbedenklichen Säureadditionssalze derselben, dadurch gekennzeichnet, daß Rosaramicin oder 12,13-Desepoxy-12,13-dehydrosaramicin mit einer Verbindung der allgemeinen Formel

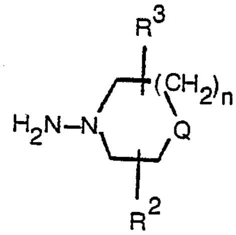

oder

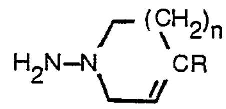

IIIa IIIb

in der n, Q, R, $R^2$ und $R^3$ die in den Ansprüchen 1 bis 10 angegebene Bedeutung haben, umgesetzt wird und die so erhaltene Verbindung in freier Form oder in Form ihrer pharmazeutisch unbedenklichen Säureadditionssalze isoliert wird.

12. Verbindung der allgemeinen Formel (I) nach einem der Ansprüche 1 bis 10 oder erhalten mittels der Verfahrens nach Anspruch 11 zur Verwendung bei der Behandlung von Infektionen.

13. Pharmazeutische Zusammensetzung, enthaltend als Wirkstoff wenigstens eine Verbindung der allgemeinen Formel (I) nach einem der Ansprüche 1 bis 10 oder erhalten mittels des Verfahrens nach Anspruch 11 zusammen mit einem pharmazeutisch unbedenklichen Träger.

**Revendications**

1. Composé de formule générale:

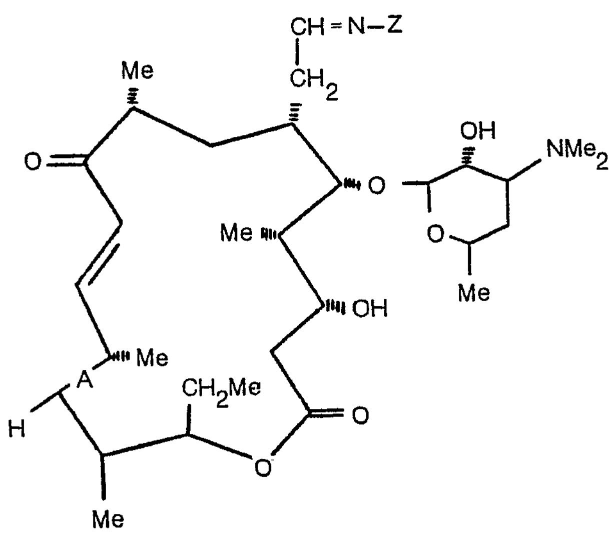

où Me est méthyle;
A représente une double liaison entre les atomes de carbone des positions 12 et 13 ou, avec les atomes de carbone des positions 12 et 13, un noyau d'oxirane;
Z est un groupe de formule IIa ou IIb,

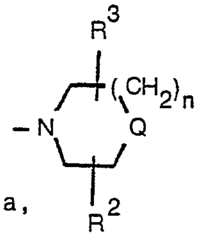

IIa,

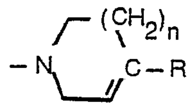

II b

où Q est $CRR^1$, NR, O, S, $SO_2$,

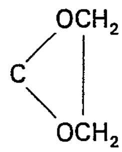

,

$CROR^1$, $CROC(O)R^1$, CHCOOR, $CHCONRR^1$ ou CRCN; où R et $R^1$ représentent indépendamment l'un de l'autre de l'hydrogène, un alcoyle contenante de 1 à 8 atomes de carbone, un aralcoyle contenant 7 à 13 atomes de carbone ou un aryle contenant de 6 à 10 atomes de carbone, les groupes aryles étant non substitués ou étant substitués par un halogène, un trifluorométhyle, ou un alcoxy contenant 1 à 5 atomes de carbone ou un alkyl carbonyle contenant 1 à 5 atomes de carbone ou un alcoyle contenant 1 à 5 atomes de carbone et quand l'un de R et $R^1$ est de l'hydrogène, l'autre peut également être un hydroxyalcoyle contenant 1 à 8 atomes de carbone; $R^2$ et $R^3$ sont indépendamment l'un de l'autre de l'hydrogène, un alcoyle contenant 1 à 8 atomes de carbone, oxo ou thioxo;
n est zéro, 1 ou 2;
ou un sel d'addition d'acide acceptable en pharmacie.

2. Composé de formule I selon la revendication 1, où Z est un groupe de formule (IIa), où $R^2$ et $R^3$ sont de l'hydrogène et Me, A, Q et n sont définis à la revendication 1.

3. Composé de formule I selon la revendication 2, où Q est $CRR^1$, NR, O, S, $SO_2$,

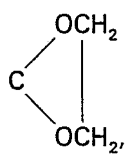

,

$CROR^1$, $CROC(O)R^1$, CHCOOR, $CHCONRR^1$ ou CRCN; où R et $R^1$ représentent indépendamment l'un de l'autre de l'hydrogène, un alcoyle contenant 1 à 5 atomes de carbone, un aralcoyle contenant 7 à 13 atomes de carbone ou un aryle contenant 6 atomes de carbone, les groupes aryles étant non substitués ou étant substitués par un fluoro, chloro, trifluorométhyle, méthoxy, acétyle et méthyle et quand l'un de R et $R^1$ est de l'hydrogène, l'autre peut également être un hydroxyalcoyle contenant 1 ou 2 atomes de carbone.

4. Composé de formule I selon la revendication 3, où n est zéro ou 1.

5. Composé de formule I selon la revendication 3, où Me et A sont définis à la revendication 1, Z est un groupe de formule (IIa), où $R^2$ et $R^3$ sont de l'hydrogène, Q est $NCH_3$, S, $SO_2$,

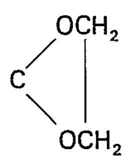

ou CHOH et n est un.

6. Composé de formule I selon la revendication 3, qui est
20-[(1,1-dioxothiomorpholino)imino]-20-désoxyrosaramicine,
20-[(4-hydroxypipéridino)imino]-20-désoxyrosaramicine,
20-[(pipéridino)imino]-20-désoxyrosaramicine,
20-[(4,4-éthylènedioxypipéridino)imino]-20-désoxyrosaramicine,
20-[(4-méthoxypipéridino)imino]-20-désoxyrosaramicine,
20-[(4-acétoxypipéridino)imino]-20-désoxyrosaramicine,
20-[(4-carboxypipéridino)imino]-20-désoxyrosaramicine,
20-[(4-diméthylaminocarbonylpipéridino)imino]-20-désoxyrosaramicine,
20-[(4-méthyl-1-pipérazinyl)imino]-20-désoxyrosaramicine,

20-[(morpholino)imino]-20-désoxyrosaramicine,
20-[(thiomorpholino)imino]-20-désoxyrosaramicine,
20-[(4-carbamoylpipéridino)imino]-20-désoxyrosaramicine,
20-[(1-pyrrolidinyl)imino]-20-désoxyrosaramicine,
20-[(1-homopipéridyl)imino]-20-désoxyrosaramicine,
20-[(1-pipérazinyl)imino]-20-désoxyrosaramicine,
12,13-désépoxy-12,13-déhydro-20-[(1-pipérazinyl)imino]-20-désoxyrosaramicine,
12,13-désépoxy-12,13-déhydro-20-[(4-méthyl-1-pipérazinyl)imino]-20-désoxyrosaramicine,
12,13-désépoxy-12,13-déhydro-20-[(morpholino)imino]-20-désoxyrosaramicine,
12,13-désépoxy-12,13-déhydro-20-[(thiomorpholino)imino]-20-désoxyrosaramicine,
12,13-désépoxy-12,13-déhydro-20-[(1,1-dioxothiomorpholino)imino]-20-désoxyrosaramicine,
12,13-désépoxy-12,13-déhydro-20-[(4,4-éthylènedioxypipéridino)imino]-20-désoxyrosaramicine,
12,13-désépoxy-12,13-déhydro-20-[(4-hydroxypipéridino)imino]-20-désoxyrosaramicine,
12,13-désépoxy-12,13-déhydro-20-[(4-éthoxypipéridino)imino]-20-désoxyrosaramicine,
12,13-désépoxy-12,13-déhydro-20-[(4-benzoyloxypipéridino)imino]-20-désoxyrosaramicine,
12,13-désépoxy-12,13-déhydro-20-[(4-propyl-4-hydroxypipéridino)imino]-20-désoxyrosaramicine,
12,13-désépoxy-12,13-déhydro-20-[(4-méthyl-4-n-butoxypipéridino)imino]-20-désoxyrosaramicine,
12,13-désépoxy-12,13-déhydro-20-[(4-pentyl-4-propionyloxypipéridino)imino]-20-désoxyrosaramicine,
12,13-désépoxy-12,13-déhydro-20-[(4-carboxypipéridino)imino]-20-désoxyrosaramicine,
20-[(4-éthyl-4-hydroxypipéridino)imino]-20-désoxyrosaramicine,
20-[(4-éthyl-4-méthoxypipéridino)imino]-20-désoxyrosaramicine,
20-[(4-méthyl-4-propionyloxypipéridino)imino]-20-désoxyrosaramicine,
20-[(4-méthoxycarbonylpipéridino)imino]-20-désoxyrosaramicine,
12,13-désépoxy-12,13-déhydro-20-[(pipéridino)imino]-20-désoxyrosaramicine,
12,13-désépoxy-12,13-déhydro-20-[(4-méthoxycarbonylpipéridino)imino]-20-désoxyrosaramicine,
12,13-désépoxy-12,13-déhydro-20-[(4-carbamoylpipéridino)imino]-20-désoxyrosaramicine, et
12,13-désépoxy-12,13-déhydro-20-[(diméthylaminocarbonylpipéridino)imino]-20-désoxyrosaramicine.

7. Composé de formule I selon la revendication 1, où Z est un groupe de formule (IIa), Q est $CH_2$, $R^2$ et $R^3$ sont indépendamment l'une de l'autre au alcoyle contenant de 1 à 8 atomes de carbone, oxo ou thioxo, n est zéro ou un et Me et A sont définis à la revendication 1.

8. Composé selon la revendication 7, qui est
20-[(2,6-diméthylpipéridino)imino]-20-désoxyrosaramicine,
20-[(4-oxo-2-thioxo-3-thiazolidinyl)imino]-20-désoxyrosaramicine, ou
20-[(2,4-dioxo-1-imidazolidinyl)imino]-20-désoxyrosaramicine.

9. Composé de formule I selon la revendication 1, où Z est un groupe de formule (IIb), n est un et Me, A et R sont tels que définis à la revendication 1.

10. Composé selon la revendication 9, qui est 20-{[(4-p-chlorophényl)-3,4-dehydropipéridino]-imino}-20-désoxyrosaramicine ou
20-[(4-phényl-3,4-déhydropipéridino)imino]-20-désoxyrosaramicine.

11. Procédé pour la préparation de composés de formule générale I selon la revendication 1 et leurs sels d'addition d'acide acceptables en pharmacie, caractérisé en ce que l'on fait réagir de la rosaramicine ou de la 12,13-désépoxy-12,13-déshydrorosaramicine avec un composé de formule générale

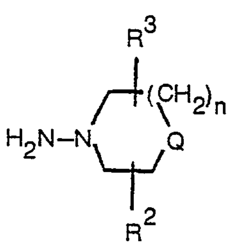
IIIa

ou

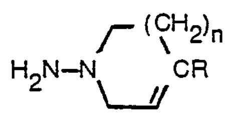
IIIb

où n, Q, R, $R^2$ et $R^3$ sont définis selon l'une quelconque des revendications 1 à 10 et le composé ainsi obtenu est isolé sous forme libre ou sous la forme de ses sels d'addition d'acide acceptables en pharmacie.

12. Composé de formule générale (I) selon l'une quelconque des revendications 1 à 10 ou obtenu par le procédé selon la revendication 11 pour traiter les infections.

13. Composition pharmaceutique comprenant comme ingrédient actif au moins un composé de formule générale (I) tel que défini selon l'une quelconque des revendications 1 à 10 ou obtenu par un procédé selon la revendication 11 avec un véhicule acceptable en pharmacie.